# EUROPEAN PATENT APPLICATION

(11) **EP 3 025 646 A1**
(43) Date of publication of application: **01.06.2016**
(21) Application number: 15195357.7
(22) Date of filing: 19.11.2015
(51) Int. Cl.: A61B 5/1455, A61B 5/00

(54) **BIOLOGICAL INFORMATION MEASURING DEVICE**

(30) Priority: 28.11.2014 JP 2014241442
(71) Applicant: Konica Minolta, Inc., Tokyo 100-7015 (JP)
(72) Inventor: Tateda, Norihiro, Tokyo, 100-7015 (JP); Kawada, Kenji, Tokyo, 100-7015 (JP); Kameda, Masanobu, Tokyo, 100-7015 (JP); Araki, Ryosuke, Tokyo, 107-0052 (JP)
(74) Representative: Hoffmann Eitle

(57) **Abstract**

A biological information measuring device includes a first holding section containing a first measuring portion that is a portion of a measuring section for measuring biological information, a second holding section containing a second measuring portion that is another portion of the measuring section, a communication section having a communication chamber allowing the first holding section to communicate with the second holding section, and a connecting portion placed in the communication chamber to electrically connect the first measuring portion with the second measuring portion.

## Description

### Technical Field

The present invention relates to a biological information measuring device for detecting various types of biological information from a finger of a living body in a non-invasive manner.

### Background Art

Various biological information measuring devices for measuring predetermined physiological information (biological information) on living bodies are known in order to determine conditions of the living bodies. A sphygmograph measures pulse waves of a living body, for example. A pulse oximeter measures a blood oxygen saturation level and a heart rate of a living body, for example. A cardiac output flowmeter measures a cardiac output. A blood glucose meter, for example, measures a blood glucose level of a living body. To measure such biological information, a photoelectric technique is employed as one of techniques for measuring biological information. A biological information measuring device using the photoelectric technique measures predetermined biological information by, for example irradiating a finger of a living body and receiving light reflected therefrom. When such a biological information measuring device receives water, water will enter the device, resulting in the possibility of a mechanical failure. To prevent this, the biological information measuring device needs to be waterproof.

For example, Japanese Unexamined Patent Publication No. 2009-504253 (hereinafter, referred to as "First Publication") discloses a probe which includes a light-emitting unit and a light-receiving sensor and is waterproofed with a cover member. In Japanese Unexamined Patent Publication No. 2009-504253, a device main body part separated from the probe and connected to the probe by a cable calculates metrics based on data obtained by the probe.

For example, Japanese Unexamined Patent Publication No. 2007-167184 (hereinafter, referred to as "Second Publication") discloses a biological information measuring device which includes a first holding section provided with a portion of a measuring section and a second holding section provided with another portion of the measuring section and in which these portions of the measuring section are electrically connected to each other, and the first holding section and the second holding section are rotatably coupled to each other. A finger is placed on a finger placement part between the first holding section and the second holding section and sandwiched therebetween to measure biological information with the first portion of the measuring section disposed in the first holding section and the second portion of the measuring section disposed in the second holding section.

In the First Publication, however, the probe is waterproofed with the cover member, but the device main body part for calculating metrics is separated from the probe, and thus, the entire device is not waterproofed. Accordingly, when the device main body part receives water, water will enter the device main body part, resulting in the possibility of a failure in the device main body part.

In the Second Publication, since the device is not waterproofed, when the first holding section or the second holding section receives water, water enters the first holding section or the second holding section, resulting in the possibility of a failure.

In this case, it is conceivable to cover a probe with a cover member to make the entire device waterproofed in a manner similar to that disclosed in the First Publication. However, the portion of the measuring section disposed in the first holding section and the portion of the measuring section disposed in the second holding section need to be enclosed with a cover member. However, the first holding section and the second holding section are allowed to rotate during measurement. Accordingly, under the condition that the connection portion is enclosed with the cover member, smooth rotation of the first holding section and the second holding section is likely to be hindered. For this reason, it is difficult to apply the cover member described in the First Publication to the first holding section and the second holding section of the Second Publication.

### Summary of the Invention

The present invention has been made in view of the foregoing problems, and has an object of providing a biological information measuring device that is entirely waterproofed with a simple configuration.

A biological information measuring device according to the present invention comprises a first holding section containing a first measuring portion that is a portion of a measuring section for measuring biological information, a second holding section containing a second measuring portion that is the other portion of the measuring section, a communication section including a communication chamber allowing the first holding section to communicate with the second holding section, and a connecting portion placed in the communication chamber to electrically connect the first measuring portion with the second measuring portion. Thus, the biological information measuring device can have the measuring section which is entirely waterproofed with a simple configuration.

These and other objects, features and advantages of the present invention will become more apparent upon reading the following detailed description along with the accompanying drawings.

### Brief Description of Drawings

FIG. 1 is a perspective view illustrating a biological information measuring device according to an embodiment of the present invention.
FIG. 2 is a back view of the biological information measuring device illustrated in FIG. 1.
FIG. 3 is a cross-sectional view of the biological information measuring device illustrated in FIG. 1.
FIG. 4A is a side view of a first cover member used in the biological information measuring device illustrated in FIG. 1.
FIG. 4B is a plan view of the first cover member.
FIG. 4C is a bottom view of the first cover member.
FIG. 4D is a front view of the first cover member.
FIG. 5A is a side view of a second cover member used in the biological information measuring device illustrated in FIG. 1.
FIG. 5B is a plan view of the second cover member.
FIG. 5C is a bottom view of the second cover member.
FIG. 5D is a front view of the second cover member.
FIG. 6A is a side view illustrating a state in which the first cover member is engaged with the second cover member.
FIG. 6B is a front view illustrating the state in which the first cover member is engaged with the second cover member.
FIG. 7 is a block diagram of configurations of the biological information measuring device illustrated in FIG. 1 and a charging device therefor.
FIG. 8 is a perspective view illustrating a state in which the biological information measuring device illustrated in FIG. 1 is charged by the charging device.

### Description of Embodiments

An embodiment of the present invention will be described with reference to the drawings. The same components are designated by the same reference characters, and description thereof is not repeated when unnecessary. Numerals without suffixes herein collectively identify corresponding parts, and numerals with suffixes individually identify corresponding components.

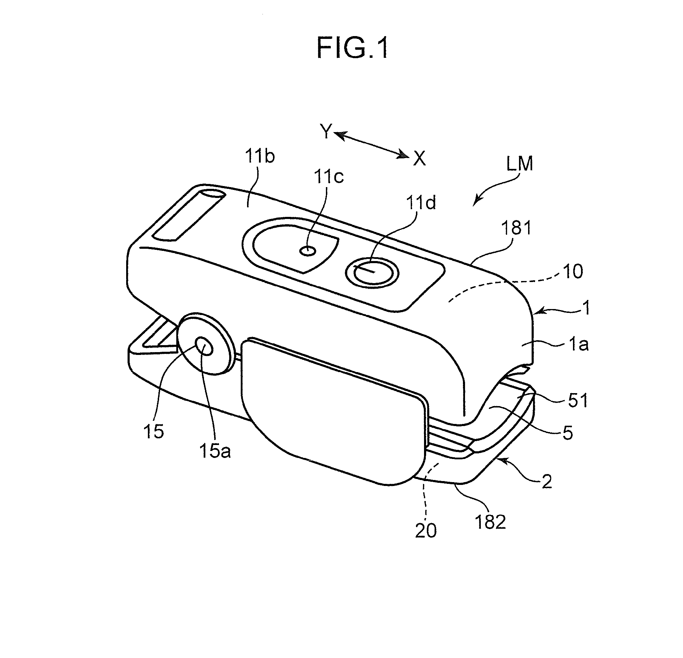

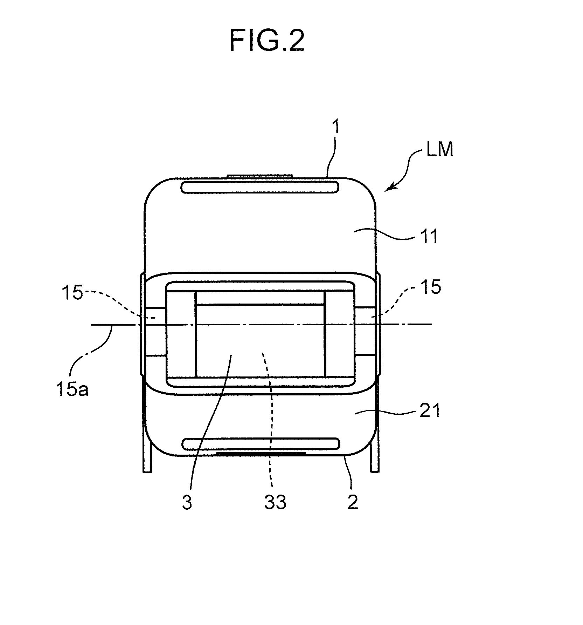

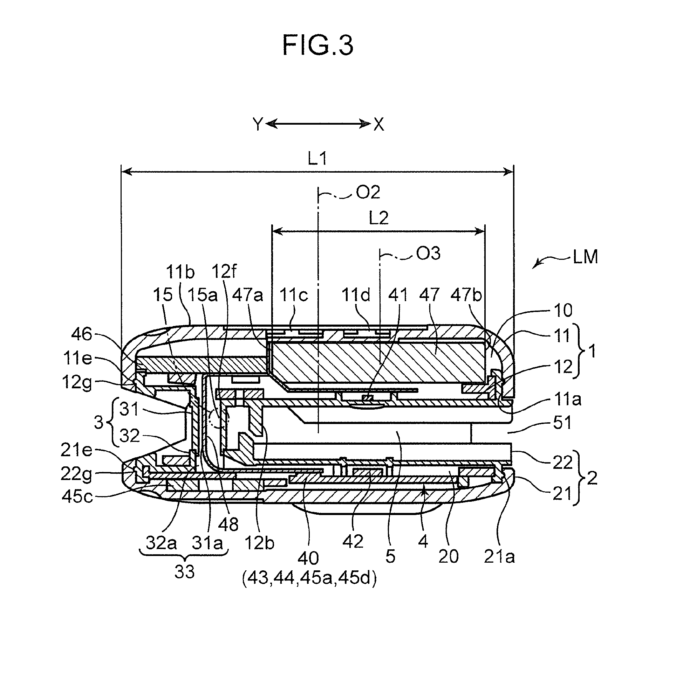

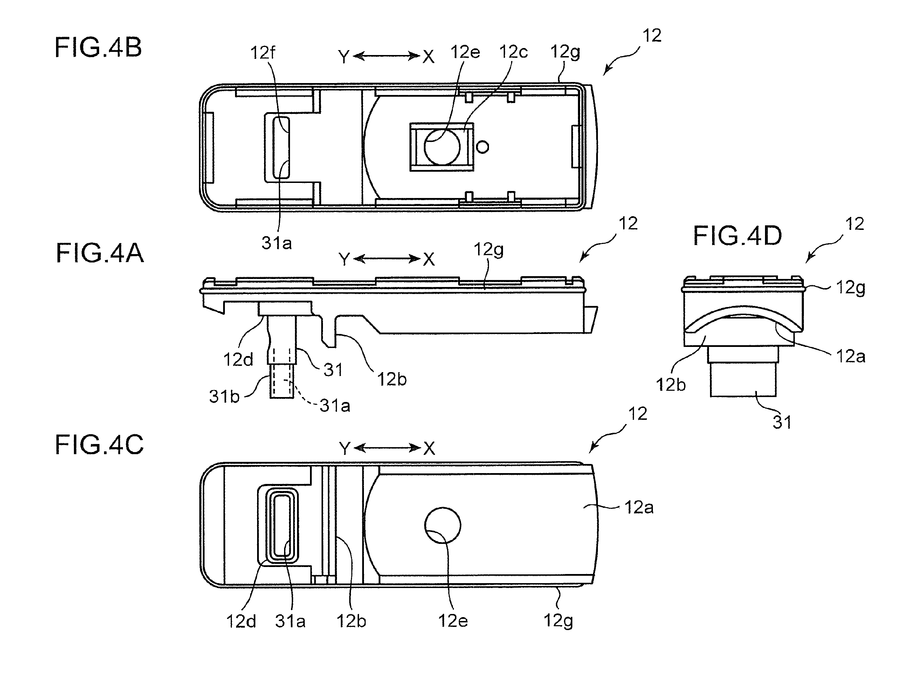

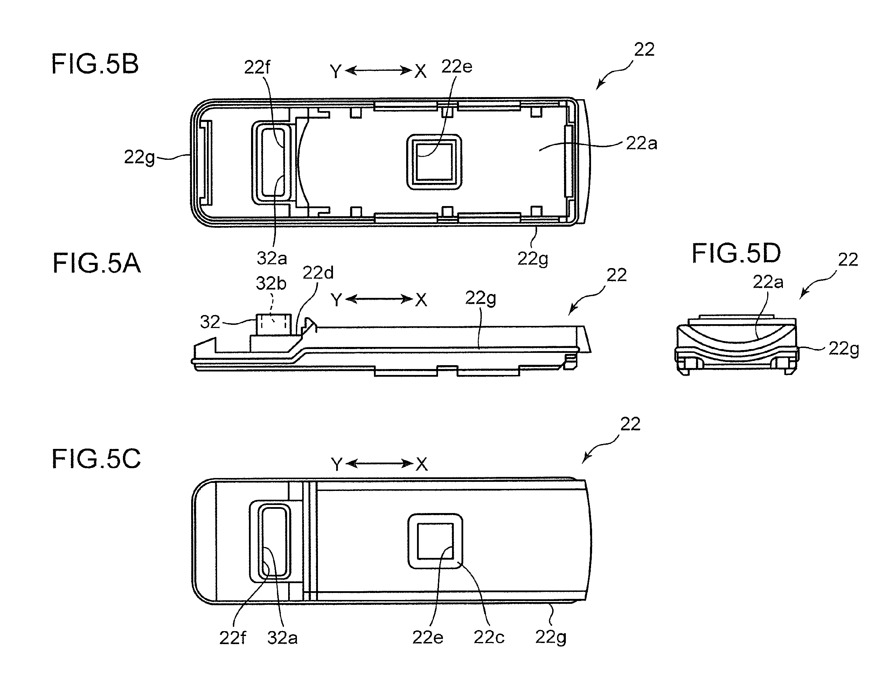

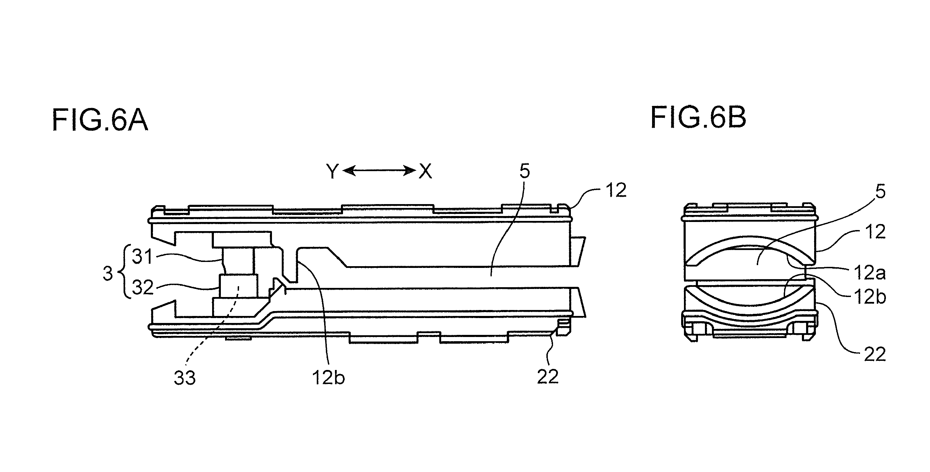

FIG. 1 is a perspective view illustrating an appearance of a biological information measuring device according to an embodiment. FIG. 2 is a back view of the biological information measuring device illustrated in FIG. 1. FIG. 3 is a cross-sectional view of the biological information measuring device illustrated in FIG. 1. FIG. 4A is a side view of a first cover member used in the biological information measuring device illustrated in FIG. 1. FIG. 4B is a plan view of the first cover member, FIG. 4C is a bottom view of the first cover member, and FIG. 4D is a front view of the first cover member. FIG. 5A is a side view of a second cover member used in the biological information measuring device illustrated in FIG. 1. FIG. 5B is a plan view of the second cover member, FIG. 5C is a bottom view of the second cover member, and FIG. 5D is a front view of the second cover member. FIG. 6A is a side view illustrating a state in which the first cover member is engaged with the second cover member. FIG. 6B is a front view of the state illustrated in FIG. 6A.

The biological information measuring device according to the embodiment is a device for measuring predetermined physiological information (biological information) from a living body when attached to a part of the living body. More specifically, in the present embodiment, a biological information measuring device LM is a photoelectric device that is attached to a finger (e.g., a maniphalanx) of a living body, irradiates the finger of the living body with a predetermined measuring light beam, receives a detection light beam derived from the measuring light beam and coming from the inside of the finger, and based on the light-reception result, obtains predetermined biological information. Examples of the predetermined biological information include a pulse wave, a blood oxygen saturation level, a pulse rate, a cardiac output, and a blood glucose level of the living body.

As illustrated in FIGS. 1 to 3, the biological information measuring device LM according to the present embodiment includes a first housing 1 in which a substantially closed first holding section 10 is defined, a second housing 2 in which a substantially closed second holding section 20 is defined, a communication section 3 including a communication chamber 33 that allows the first holding section 10 to communicate with the second holding section 20, and a measuring section 4 for measuring biological information.

The first housing 1 includes a first housing main body 11 and a first cover member 12. The first housing main body 11 is substantially cuboid, and includes a first opening 11a facing the second housing 2.

The first housing main body 11 includes a first engaging recess 11e with which the first cover member 12 is engaged. The first engaging recess 11e is recessed from an entire inner circumferential surface of the first housing main body 11 near the first opening 11a.

The first housing main body 11 includes an upper wall surface 11b facing the first opening 11a, and also includes a display unit 11c and an operation unit 11d on an upper surface 11b. The display unit 11c is a unit for displaying an operating state of the biological information measuring device LM, and is constituted by, for example, an LED in the present embodiment. The LED as the display unit 11c displays an operating state of the biological information measuring device LM by changing the color (e.g., red, green, or blue) of emitted light and a lighting state (e.g., continuous lighting or blinking). In the present embodiment, the operating states includes a measuring state that the device is in the measurement operation, a charging state that the device is in the process of being charged, and a charging completed state that the charging of the device has been completed. The display unit 11c may be, for example, a display device such as an LCD or an organic EL display in order to display the type of operation input from the operation unit 11d or a measurement result (e.g., a blood oxygen saturation level).

The operation unit 11 d is, for example, a unit for inputting a predetermined operation to the biological information measuring device LM, and may be composed of one or more input switches each assigned with a predetermined function, for example. More specifically, in the present embodiment, the operation unit 11d is a push-bottom power switch for turning on a power supply to start measurement.

In the present embodiment, the first cover member 12 is composed of an elastic synthetic resin, and as illustrated in FIGS. 4A to 4D, has a rectangular shape substantially the same as those of the first opening 11a of the first housing main body 11 in plan view so as to cover the entire first opening 11a.

The first cover member 12 includes a first finger placement recess 12a, a fingertip touching part 12b, an irradiation part holder 12c that holds an irradiation part 41(see FIG. 3) of the measuring section 4 described later, a first communication section holder 12d that holds a first communication section 31 of the communication section 3 described later, and a first cover member engaging projection 12g for snugly fitting the first cover member 12 in the first housing main body 11.

The first finger placement recess 12a and a second finger placement recess 22a of the second cover member 22 described later together form the finger placement part 5. The first finger placement recess 12a is recessed upward to form a curve (an arch) in cross section from one end face to the other end face of the first cover member 12 in a longitudinal direction (X-Y direction) to the fingertip touching part 12b so that a back part of a finger, for example, can be contained therein.

The fingertip touching part 12b is used to always restrict the placement extent of the finger on the finger placement part 5 to a constant extent by coming into contact with a finger placed on the finger placement part 5. The fingertip touching part 12b projects toward the second housing 2 below the lower surface (one surface in the thickness direction) of the first cover member 12.

The irradiation part holder 12c is disposed substantially at a center of the first finger placement recess 12a in the longitudinal direction on the upper surface (the other surface in the thickness direction) of the first cover member 12.

The irradiation part holder 12c includes a circular irradiation part exposing hole 12e penetrating the first cover member 12 from the upper surface to the lower surface thereof.

The first communication section holder 12d is located at the side of the fingertip touching part 12b opposite to the first finger placement recess 12a in the longitudinal direction of the first cover member 12. The first communication section holder 12d includes a rectangular through hole 12f penetrating the first cover member 12 from the upper surface to the lower surface thereof.

The first cover member engaging projection 12g projects sideways from the entire outer circumference of the first cover member 12.

As illustrated in FIG. 3, the thus-configured first cover member 12 includes a first engaging projection 12g that is fitted in the first engaging recess 11e of the first housing main body 11 so that the first cover member 12 is assembled to the first housing main body 11. In this assembly state, the first cover member 12 and the first housing main body 11 define the substantially closed first holding section 10.

In a manner similar to the first housing 1, as illustrated in FIG. 3, the second housing 2 includes a second housing main body 21 and a second cover member 22. The second housing main body 21 is substantially cuboid, and includes a second opening 21 a facing the first housing 1.

The second housing main body 21 includes a second engaging recess 21e with which the second cover member 22 is engaged. The second engaging recess 21e is recessed from an entire inner circumferential surface of the second housing main body 21 near the second opening 21a.

As illustrated in FIGS. 5A to 5D, the second cover member 22 has substantially the same shape as that of the cover member 12, and has substantially the same rectangular shape as that of the second opening 21 a of the second housing main body 21 in cross section so as to cover the entire second opening 21 a.

The second cover member 22 includes a second finger placement recess 22a, a light-receiving part holder 22c that holds a light-receiving part 42 of the measuring section 4 described later, a second communication section holder 22d that holds a second communication section 32 of the communication section 3 described later, and a second cover member engaging projection 22g for engaging the second cover member 22 with the second housing main body 21.

As described above, the second finger placement recess 22a and the first finger placement recess 12a of the first cover member 12 together form the finger placement part 5. The second finger placement recess 22a is recessed downward in the direction opposite to the first finger placement recess 12a to form a curve (an arch) in cross section at the position opposite to the first finger placement recess 12a from the upper surface (one surface in the thickness direction) of the second cover member 22 opposite to the first cover member 12 so that a finger pad side is contained in the second finger placement recess 22a, for example.

The light-receiving part holder 22c is disposed substantially at a center of the second finger placement recess 22a in the longitudinal direction on the lower surface (the other surface in the thickness direction) of the second cover member 22.

The light-receiving part holder 22c includes a rectangular light-receiving part exposing hole 22e penetrating the second cover member 22 from the upper surface to the lower surface thereof at a position facing the irradiation part exposing hole 12e.

The second communication section holder 22d is disposed at a position facing the first communication section holder 12d of the first cover member 12. The second communication section holder 22d includes a rectangular through hole 22f penetrating the second cover member 22 from the upper surface to the lower surface thereof.

As illustrated in FIG. 3, the thus-configured second cover member 22 includes a second engaging projection 22g that is fitted in the second engaging recess 21e of the second housing main body 21 so that the second cover member 22 is assembled to the second housing main body 21. In this assembly state, the second cover member 22 and the second housing main body 21 define the substantially closed second holding section 20.

As described above, the second housing 2 to which the second cover member 22 and the second housing main body 21 are assembled and the first housing 1 to which the first cover member 12 and the first housing main body 11 are assembled are coupled to each other in such a manner that the second housing 2 and the first housing 1 can rotate relative to each other.

More specifically, as illustrated in FIGS. 1 to 3, with the first cover member 12 and the second cover member 22 being opposed to each other at a predetermined distance, side walls of a rear end portion (the other end portion) of the first housing main body 11 in the longitudinal direction (X-Y direction) and side walls of a rear end portion (the other end portion) of the second housing main body 21 in the longitudinal direction are pivotably supported by a shaft member 15. The first housing 1 and the second housing 2 are rotatable around a lateral rotation axis formed by the shaft member 15.

By coupling the first housing 1 and the second housing 2 with each other, the first finger placement recess 12a and the second finger placement recess 22a define the finger placement part 5 therebetween.

In the present embodiment, the finger placement part 5 is shaped in such a manner that a finger is inserted thereinto through a finger insertion opening 51 formed in end faces 1a of the first housing 1 and the second housing 2 at one end in the longitudinal direction. The finger placement part 5 extends toward end faces of the first housing 1 and the second housing 2 at the other end in the longitudinal direction to reach the fingertip touching part 12b. The direction in which the finger is inserted into the finger placement part 5 is a direction (X-Y direction) along the longitudinal direction of the first housing 1 and the second housing 2.

In regard to the finger placement part 5, when the rear end portions (the other end portions) of the first housing 1 and the second housing 2 are pressed to approach each other, one of the first housing 1 or the second housing 2 rotates relative to the other around the rotation axis formed by the shaft member 15, thereby enlarging the finger insertion opening 51. When the pressing force is removed, an unillustrated biasing member provided in the device causes the finger insertion opening 51 to return to the original state.

Then, the communication section 3 will be described. As illustrated in FIGS. 3 to 6, the communication section 3 is made of an elastic material, and includes a first communication section 31 in the shape of a rectangular prism having a first communication chamber 31a and a second communication section 32 in the shape of a rectangular prism having a second communication chamber 32a.

As illustrated in FIGS. 4A to 4D, an upper end (one end) of the first communication section 31 is connected to the first communication section holder 12d of the first cover member 12 in such a manner that the first communication chamber 31a coincides with the through hole 12f. In the present embodiment, the first communication section 31 is made of the same material as the first cover member 12, and is integrated with the first cover member 12. Thus, the one end (upper end) of the first communication section 31 is connected to the first communication section holder 12d of the first cover member 12. A lower end (the other end) of the first communication section 31 protrudes from the first cover member 12 toward the second cover member 22, and an engaging projection (engaging part) 31b is provided on an outer periphery of the tip of the protrusion thereof.

As illustrated in FIGS. 5 to 5D, the second communication section 32 extends in such a manner that an upper end (one end) thereof protrudes from the second cover member 22 upward, i.e., toward the first cover member 12. An engaged recess (engaged part) 32b that is detachably engaged with the engaging projection 31b is provided on the inner periphery of the tip of the protraction thereof. A lower end (the other end) of the second communication section 32 is connected to the second communication section holder 22d of the second cover member 22 in such a manner that the second communication chamber 32a coincides with the through hole 22f.

In the present embodiment, the second communication section 32 is made of the same material as the second cover member 22, and is integrated with the second cover member 22. Thus, the lower end of the second communication section 32 is connected to the second communication section holder 22d of the second cover member 22.

As illustrated in FIG. 3 and FIGS. 6A and 6B, the engaging projection 31b of the first communication section 31 is engaged with the engaged recess 32b of the second communication section 32 so that the first communication chamber 31a communicates with the second communication chamber 32a. In this manner, one communication section 3 extending from the first housing 1 to the second housing 2 is defined by the first communication section 31 and the second communication section 32 between the first housing 1 and the second housing 2. The first holding section 10 of the first housing 1 communicates with the second holding section 20 of the second housing 2 through the communication chamber 33.

The communication section 3 is disposed in such a manner that an axial center 15a of the rotation axis of the first housing 1 and the second housing 2 perpendicularly intersects the direction in which the communication section 3 extends, and passes through the communication section 3.

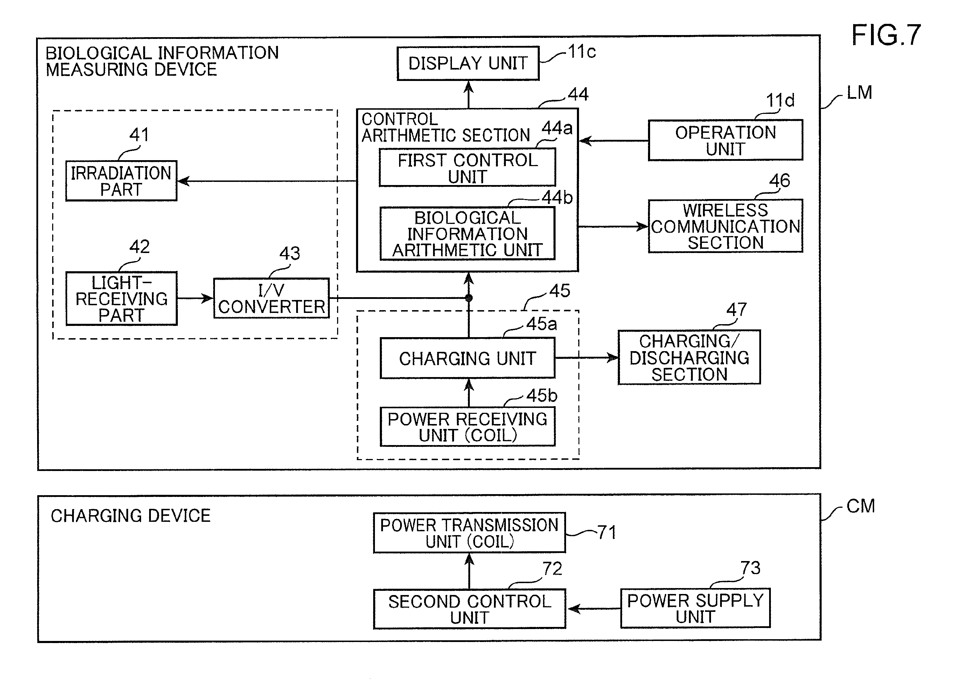

Then, the measuring section 4 will be described. FIG. 7 is a block diagram of configurations of the biological information measuring device illustrated in FIG. 1 and a charging device therefor. As illustrated in FIGS. 3 and 7, the measuring section 4 includes measuring units 41, 42, and 43, a control arithmetic section 44, a power receiving charging section 45, a wireless communication section 46, a charging/discharging section 47, and a connecting portion 48.

The measuring units 41, 42, and 43 are connected to the control arithmetic section 44, and measure predetermined biological information based on control of the control arithmetic section 44. The measuring units 41, 42, and 43 output the measurement result to the control arithmetic section 44. Each of the measuring units 41, 42, and 43 employs specific known configurations for biological information to be measured. In the present embodiment, a blood oxygen saturation level (SpO₂) is measured with a photoelectric technique, and the measuring unit includes the irradiation part 41, the light-receiving part 42, and a current-to-voltage converter (I/V converter) 43.

The irradiation part 41 is connected to the control arithmetic section 44 and applies a measuring light beam with a predetermined wavelength based on control of the control arithmetic section 44. The irradiation part 41 is held by the irradiation part holder 12c of the first cover member 12 and is contained in the first holding section 10.

The light-receiving part 42 is connected to the I/V converter 43, receives a detection light beam, and outputs a result of the received light beam (a current obtained by photoelectric conversion of the detection light beam) to the I/V converter 43. The detection light beam is obtained by passing the measuring light beam through part of a living body to be measured, such as a maniphalanx, or by reflecting the measuring light beam on the part (which is the maniphalanx in this example) of the living body. Thus, the irradiation part 41 and the light-receiving part 42 are disposed in an appropriate layout (e.g., opposed layout or separated parallel layout) so as to utilize a transmitted detection light beam or an appropriate layout (e.g., adjacent parallel layout) so as to utilize a reflected detection light beam. In the biological information measuring device LM according to the present embodiment, the irradiation part 41 and the light-receiving part 42 are opposed to each other at a predetermined distance so as to utilize a transmitted detection light beam (opposed layout).

More specifically, the light-receiving part 42 is held by the light-receiving part holder 22c of the second cover member 22 and is contained in the second holding section 20.

The measuring light beam is a light beam with a wavelength with which the predetermined biological information, a blood oxygen saturation level in the present embodiment, can be detected. More specifically, in the case of detecting a blood oxygen saturation level by using a difference in absorbance in accordance with the wavelength between hemoglobin and oxyhemoglobin, the measuring light beam is, for example, a red light beam or an infrared light beam. The irradiation part 41 includes, for example, an irradiation optical system such as a light source, e.g., a light-emitting diode (LED), and a peripheral circuit thereof such as a driving circuit. The light-receiving part 42 includes a light-receiving optical system such as a photoelectric conversion element, e.g., a silicon photodiode, and a peripheral circuit thereof. In this manner, the irradiation part 41 and the light-receiving part 42 constitute an irradiating/light-receiving part that irradiates a part of the living body with a predetermined measuring light beam and receives a detection light beam from the part of the living body derived from the measuring light beam so that the received light beam is used for obtaining predetermined biological information.

The I/V converter 43 is placed in the second holding section 20, is connected to the control arithmetic section 44, and converts a current input from the light-receiving part 42 and generated by photoelectric conversion of a detection light beam in the light-receiving part 42 to a voltage. The obtained voltage is output from the I/V converter 43 to the control arithmetic section 44.

The control arithmetic section 44 controls the units of the biological information measuring device LM in accordance with the functions of the units. Thus, the control arithmetic section 44 controls a measuring operation of the measuring unit. The control arithmetic section 44 obtains predetermined biological information based on the light receiving result of the light-receiving part 42 through the I/V converter 43 in the measuring unit, and is contained in the second holding section 20 in the present embodiment.

The control arithmetic section 44 includes a microcomputer including a central processing unit (CPU), a memory, and peripheral circuits thereof. As illustrated in FIG. 7, a first control unit 44a and a biological information arithmetic unit 44b are functionally configured in accordance with execution of a program. The first control unit 44a controls the units of the biological information measuring device LM in accordance with the functions thereof.

The first control unit 44a stops a measuring operation of the measuring unit while the charging/discharging section 47 is being charged by the power receiving charging section 45. When the charging of the charging/discharging section 47 by the power receiving charging section 45 is finished, power supply to the first control unit 44a is turned off. The biological information arithmetic unit 44b obtains predetermined biological information based on a light reception result (photoelectric pulse wave signal) of the light-receiving part 42 in the measuring unit. A blood oxygen saturation level, that is an example of biological information, can be calculated in one of well known conventional ways.

The wireless communication section 46 is contained in the first holding section 10, is connected to the control arithmetic section 44, and wirelessly transmits a communication signal to an external device. The biological information obtained by the control arithmetic section 44 is transmitted to the wireless communication section 46 so that a structure for analyzing and storing the biological information does not need to be installed in the biological information measuring device LM. As a result, a display device such as an LCD can be omitted, or size reduction, power consumption reduction, and cost reduction of the display device can be achieved. The wireless communication section 46 may transmit the light reception result obtained by the light-receiving part 42 of the measuring unit to an external device. In this manner, the biological information arithmetic unit 44b of the control arithmetic section 44 can be omitted.

The charging/discharging section 47 is used for charging and discharging. The charging/discharging section 47 is connected to the power receiving charging section 45, and components of the biological information measuring device LM that need electric power, such as the irradiation part 41, the display unit 11c, the control arithmetic section 44, and the wireless communication section 46. The charging/discharging section 47 is charged with electric power received by the power receiving charging section 45, and supplies the electric power to the components of the biological information measuring device LM that need electric power. The charging/discharging section 47 includes a secondary battery (storage battery) and a peripheral circuit thereof, for example. The charging/discharging section 47 includes a nickel-cadmium battery, a nickel-metal hydride battery, or a lithium ion battery, for example.

In the present embodiment, the charging/discharging section 47 is contained in the first holding section 10 in such a manner that one end 47a of the charging/discharging section 47 in the finger insertion direction (X-Y direction) of the finger placement part 5 is disposed toward the finger insertion opening 51 at one end of the first housing 1 and the second housing 2 in the longitudinal direction, whereas the other end 47b of the charging/discharging section 47 in the finger insertion direction is disposed toward the other end of the first housing 1 and the second housing 2 in the longitudinal direction.

The charging/discharging section 47 is disposed in such a manner that a center 03 of a length L2 of the finger placement part 5 in the finger insertion direction, i.e., a center 03 between the one end 47a and the other end 47b in the finger insertion direction of the finger placement part 5 in the charging/discharging section 47 is closer to the finger insertion opening 51 than a center 02 of a length L1 along the longitudinal direction of the first housing 1 and the second housing 2 is.

In the present embodiment, the charging/discharging section 47 is disposed in such a manner that the one end 47a and the other end 47b are closer to the finger insertion opening 51 than the fingertip touching part 12b.

The power receiving charging section 45 is connected to the control arithmetic section 44 and the charging/discharging section 47 and charges the charging/discharging section 47 in a noncontact charging manner. The power receiving charging section 45 includes a charging unit 45a and a power receiving unit 45b, for example. The power receiving unit 45b includes a power receiving coil 45c that is a coil for generating an electromotive force from a magnetic field by electromagnetic induction and a peripheral circuit 45d constituting a resonant circuit in combination with the power receiving coil 45c. The charging unit 45a charges the charging/discharging section 47 with electric power received by the power receiving unit 45b. The power receiving coil 45c is stored in the first holding section 10.

In the present embodiment, the second holding section 20 houses a main board 40, and the main board 40 is provided with the I/V converter 43 and the control arithmetic section 44 of the measuring unit and the power receiving unit 45b and the peripheral circuit 45d of the power receiving charging section, for example.

Then, the connecting portion 48 will be described. The connecting portion 48 electrically connects a first measuring portion as a part of the measuring section 4 to a second measuring portion contained in the second holding section 20 and composed of another part of the measuring section 4.

As described above, in the present embodiment, the irradiation part 41, the wireless communication section 46, and the charging/discharging section 47 are contained in the first holding section 10, and together constitute the first measuring portion. On the other hand, as described above, the control arithmetic section 44, the I/V converter 43, the charging unit 45a, the power receiving coil 45c, and the peripheral circuit 45d are contained in the first holding section 10, and together constitute the second measuring portion.

In the present embodiment, the connecting portion 48 is composed of a flexible board and directly or indirectly connects the first measuring portion and the second measuring portion to each other. The one end of the connecting portion 48 is connected to the first measuring portion and is disposed in the first holding section 10. The other end of the connecting portion 48 is connected to the second measuring portion and is disposed in the second holding section 20. An intermediate portion of the connecting portion 48 is inserted in the communication chamber 33 of the communication section 3.

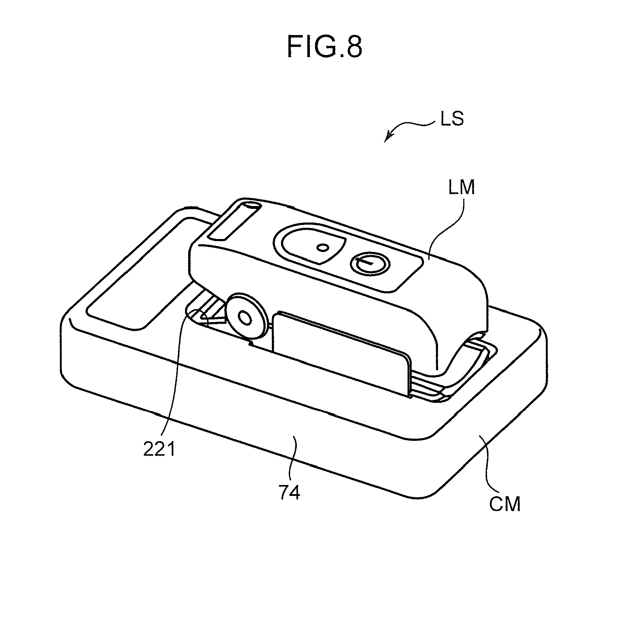

The biological information measuring device LM having the configuration described above is combined with a charging device CM for charging the biological information measuring device LM in a noncontact charging manner, thereby constituting a biological information measuring system LC. FIG. 8 is a perspective view of a state in which the biological information measuring device illustrated in FIG. 1 is charged by a charging device.

As illustrated in FIG. 7, the charging device CM includes a power transmission unit 71, a second control unit 72, and a power supply unit 73. The power transmission unit 71 transmits electric power in a noncontact charging manner, and includes a power transmission coil that is a coil for generating a magnetic field and a peripheral circuit constituting a resonant circuit in combination with the power transmission coil, for example. The second control unit 72 is connected to the power transmission unit 71 and controls operation of the power transmission unit 71. The power supply unit 73 converts, for example, commercial electric power into an electric power having a predetermined voltage value and a predetermined current value and supplies the power to the power transmission unit 71 through the second control unit 72, and is, for example, an AC adapter.

The power transmission unit 71, the second control unit 72, and the power supply unit 73 are contained in a relatively thin cuboid casing 74, as illustrated in FIG. 8. The casing 74 has a recessed portion 221 in a top surface thereof. In charging the biological information measuring device LM, the biological information measuring device LM is contained in the recessed portion 221.

Next, operation of the biological information measuring device LM will be described. To measure a blood oxygen saturation level (SpO₂) with the biological information measuring device LM, the rear end portions (the other end portions) of the first housing 1 and the second housing 2 are pressed toward each other by, for example, one hand. In this manner, in the biological information measuring device LM, one of the first housing 1 or the second housing 2 rotates relative to the other using the shaft member 15 as a rotation axis, thereby enlarging the finger insertion opening 51.

In this state, a finger of the other hand is inserted through the finger insertion opening 51 to be placed on the finger placement part 5 with the fingertip contacting the fingertip touching part 12b. When the fingertip contacts the fingertip touching part 12b, an operation of pressing the first housing 1 and the second housing 2 toward each other is stopped. In this manner, the unillustrated biasing member provided in the biological information measuring device LM causes the finger insertion opening 51 to return to the original state so that the first finger placement recess 12a of the first housing 1 and the second finger placement recess 22a of the second housing 2 pinch the finger on the finger placement part 5.

Thus, in this state, the finger on the finger placement part 5 is fixed in the biological information measuring device LM and cannot move. When the operation unit 11d is then pressed, the display unit 11c starts irradiating so that a blood oxygen saturation level (SpO₂) can be measured with the measuring section of the biological information measuring device LM.

In the thus-configured biological information measuring device LM, the first measuring portion as a portion of the measuring section is contained in the first holding section 10, the second measuring portion as another portion of the measuring section is contained in the second holding section 20, and the connecting portion 48 electrically connecting the first measuring portion to the second measuring portion is inserted into the communication chamber 33 of the communication section 3. Thus, the entire biological information measuring device LM can be made waterproof with a simple configuration.

Since the communication section 3 is composed of an elastic material, in a case where the second housing moves relative to the first housing so that a finger is placed on the finger placement part in order to measure biological information, the second housing can smoothly move relative to the first housing. In this manner, an operation of placing a finger to measure biological information can be easily performed.

The first housing main body 11 and the first cover member 12 can be made of different separate members, and the second housing main body 21 and the second cover member 22 can be made of different separate members. In this manner, the first housing 1 and the second housing 2 can be easily formed. For example, the first cover member 12 can be assembled to the first housing main body 11 in such a manner that the first measuring portion is contained in the first holding section 10 to cover the first opening with the second cover member 22 being assembled to the second housing main body 21 in such a manner that the second measuring portion is contained in the second holding section 20 to cover the second opening. In this manner, the assembly can be easily performed.

The communication section includes a cylindrical first communication section integrally formed with the first cover member, and a cylindrical second communication section integrally formed with the second cover member. The first communication section includes the engaging part at the tip of the protrusion thereof. The second communication section includes the engaged part to be engaged with the engaging part, at the tip of the protrusion thereof. Thus, the first communication section can be formed in forming the first cover member, and the second communication section can be formed in forming the second cover member. In this manner, the communication sections can be easily formed. In addition, by engaging the engaging part of the first communication section with the engaged part of the second communication section, the communication section including one communication chamber through which the first holding section communicates with the second holding section can be easily assembled.

In the present embodiment, the communication section 3 is made of an elastic material and extends from the first housing 1 toward the second housing 2. The first housing 1 and the second housing 2 are rotatably coupled to each other so as to enlarge and narrow the space of the finger placement part 5. The axial center 15a of the rotation axis of the first housing 1 and the second housing 2 perpendicularly intersects the direction in which the communication section 3 extends, and the passes through the communication section 3. In this manner, the distance from the rotation operation unit to the communication section 3 is substantially equal to the distance from the rotation operation unit to the axial center 15a of the rotation axis, a rotation torque of rotating the first housing 1 and the second housing 2 relative to each other is applied to the communication section 3 so that the first housing 1 and the second housing 2 smoothly rotate.

In the present embodiment, the first measuring portion is constituted by a portion of the measuring section, and the second measuring portion is constituted by the other portion of the measuring section. Alternatively, the second measuring portion may be made of a portion of the measuring section except the portion constituting the first measuring portion. In this manner, various modifications may be made.

In the present embodiment, the engaging part 31b of the first communication section 31 is the engaging projection, and the engaged part 32b of the second communication section 32 is the engaged recess. However, the present invention is not limited to this configuration, and the configuration may be changed in such a manner that the engaging part of the first communication section 31 is the engaged recess and the engaged part 32b of the second communication section 32 is the engaging projection.

In the present specification, techniques in various aspects have been disclosed, and major ones of these techniques are summarized below.

A biological information measuring device according to an aspect comprises: a first housing including a first holding section which is substantially closed; a second housing including a second holding section which is substantially closed; a cylindrical communication section including a communication chamber allowing the first holding section to communicate with the second holding section; and a measuring section that measures biological information. The first housing and the second housing are movably connected with each other to expandably define a finger placement part between the first and second housings to pinch a finger. The communication section is made of an elastic material. The measuring section includes a first measuring portion that is a portion of the measuring section and is contained in the first holding section, a second measuring portion that is another portion of the measuring section and is contained in the second holding section, and an connecting portion placed in the communication chamber to electrically connect the first measuring portion with the second measuring portion.

With this configuration, the first measuring portion that is a portion of the measuring section is contained in the first holding section, the second measuring portion that is the other portion of the measuring section is contained in the second holding section, and the connecting portion electrically connecting the first measuring portion to the second measuring portion is placed in the communication chamber of the communication section. Thus, the biological information measuring device can have the measuring section which is entirely waterproofed with a simple configuration.

Since the communication section is made of an elastic material, in a case where the second housing moves relative to the first housing and a finger is placed on the finger placement part in order to measure biological information, the second housing can smoothly move relative to the first housing. In this manner, an operation of placing a finger to measure biological information can be easily performed.

According to another aspect, in the biological information measuring device described above, the first housing includes a first housing main body having a first opening and a first cover member covering the first opening, the second housing includes a second housing main body having a second opening and a second cover member opposed to the first cover member and covering the second opening, and the communication section is connected with each of the first cover member and the cover member.

With this configuration, the biological information measuring device is configured in such a manner that the first housing main body and the first cover member can be made of different separate members, and the second housing main body and the second cover member can be made of different separate members. In this manner, the first housing and the second housing can be easily formed.

For example, the first cover member can be assembled to the first housing main body in such a manner that the first measuring portion is contained in the first holding section to cover the first opening with the second cover member being assembled to the second housing main body in such a manner that the second measuring portion is contained in the second holding section to cover the second opening. In this manner, the assembly can be easily performed.

According to another aspect, in the biological information measuring device described above, the communication section includes a cylindrical first communication section integrally formed with the first cover member and protruding toward the second cover member and a cylindrical second communication section integrally formed with the second cover member and protruding toward the first cover member, the first communication section includes an engaging part on a tip or leading end of the protrusion thereof, and the second communication section includes an engaged part to be engaged with the engaging part, on a tip or leading end of the protrusion thereof.

With this configuration, the first communication section can be formed when molding the first cover member, and the second communication section can be formed when molding the second cover member. In this manner, the communication section can be easily formed. In addition, by engaging the engaging part of the first communication section with the engaged part of the second communication section, the communication section including a single communication chamber through which the first holding section communicates with the second holding section can be easily assembled

This application is based on Japanese Patent application No. 2014-241442 filed in Japan Patent Office on November 28, 2014, the contents of which are hereby incorporated by reference.

Although the present invention has been fully described by way of example with reference to the accompanying drawings, it is to be understood that various changes and modifications will be apparent to those skilled in the art. Therefore, unless otherwise such changes and modifications depart from the scope of the present invention hereinafter defined, they should be construed as being included therein.

## Claims

1. A biological information measuring device comprising:
a first housing including a first holding section which is substantially closed;
a second housing including a second holding section which is substantially closed;
a cylindrical communication section including a communication chamber allowing the first holding section to communicate with the second holding section; and
a measuring section for measuring biological information; wherein
the second housing is movably connected with the first housing to expandably define a finger placement part between the first housing and the second housing,
the communication section is made of an elastic material, and
the measuring section includes a first measuring portion contained in the first holding section, a second measuring portion contained in the second holding section, and a connecting portion placed in the communication chamber to electrically connect the first measuring portion with the second measuring portion.

2. A biological information measuring device according to claim 1, wherein
the first housing includes a first housing main body having a first opening and a first cover member covering the first opening,
the second housing includes a second housing main body having a second opening and a second cover member in opposite to the first cover member and covering the second opening, and
the communication section is connected to each of the first cover member and the second cover member.

3. A biological information measuring device according to claim 2, wherein
the communication section includes a first communication portion integrally formed with the first cover member and having the shape of a cylinder protruding toward the second cover member, and a second communication portion integrally formed with the second cover member and having the shape of a cylinder protruding toward the first cover member,
the first communication portion includes an engaging section on a leading end thereof, and
the second communication portion includes an engaged section engaged with the engaging section on a leading end thereof.
